(19) Europäisches Patentamt
European Patent Office
Office européen des brevets

(11) Veröffentlichungsnummer: **0 114 607**
**B1**

(12) # EUROPÄISCHE PATENTSCHRIFT

(45) Veröffentlichungstag der Patentschrift: **04.09.85**

(51) Int. Cl.[4]: **C 07 C 49/84,** A 61 K 7/42

(21) Anmeldenummer: **84100176.1**

(22) Anmeldetag: **10.01.84**

(54) **Neue Dibenzoyl-methan-Derivate, Verfahren zu ihrer Herstellung und ihre Verwendung.**

(30) Priorität: **22.01.83 DE 3302123**

(43) Veröffentlichungstag der Anmeldung: **01.08.84 Patentblatt 84/31**

(45) Bekanntmachung des Hinweises auf die Patenterteilung: **04.09.85 Patentblatt 85/36**

(84) Benannte Vertragsstaaten: **AT CH DE FR GB IT LI NL**

(56) Entgegenhaltungen:
**CH - A - 544 052**
**DE - A - 1 959 398**
**DE - A - 2 544 180**
**DE - A - 2 945 125**

(73) Patentinhaber: **Haarmann & Reimer GmbH, Postfach 1253, D-3450 Holzminden (DE)**

(72) Erfinder: **Hopp, Rudolf, Dr., Auf dem Gehrenkamp 28, D-3450 Holzminden (DE)**
Erfinder: **Finkelmeier, Horst, Dr., Gehrenkamp 6, D-3450 Holzminden (DE)**
Erfinder: **Langner, Roland, Jahnstrasse 9, D-3454 Bevern (DE)**

(74) Vertreter: **Mann, Volker, Dr. et al, c/o Bayer AG Konzernverwaltung RP Patentabteilung, D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 114 607 B1

**Beschreibung**

Die Erfindung betrifft neue Dibenzoyl-methan-Derivate, Verfahren zu ihrer Herstellung und ihre Verwendung in Lichtschutzmitteln.

Es wurden neue Dibenzoyl-methan-Derivate der Formel

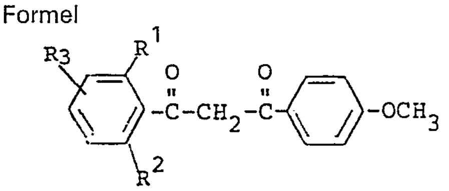
(I),

in der

$R^1$ Wasserstoff, Methyl oder Ethyl und

$R^2$ Methyl oder Ethyl bedeutet und

$R^3$ für einen niederen geradkettigen oder verzweigten Alkylrest steht,

gefunden.

Der niedere geradkettige oder verzweigte Alkylrest enthält im allgemeinen 1 bis etwa 6 Kohlenstoffatome. Beispielsweise seien die folgenden niederen Alkylreste genannt: Methyl, Ethyl, Propyl, Isopropyl, Butyl, Isobutyl, tert.-Butyl, Pentyl, Isopentyl, Hexyl und Isohexyl.

Bevorzugte neue Dibenzoyl-methan-Derivate sind Verbindungen der Formel

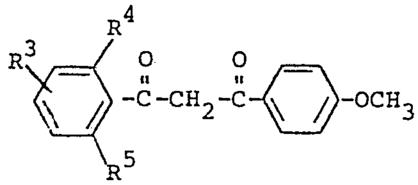
(II),

in der

$R^3$ die oben genannte Bedeutung hat,

$R^4$ Wasserstoff oder Methyl und

$R^5$ Methyl bedeutet.

Im einzelnen seien die folgenden neuen Dibenzoyl-methan-Derivate genannt: 2-Methyl-5-isopropyl-4'-methoxy-dibenzoyl-methan, 2-Methyl-5-tert.-butyl-4'-methoxy-dibenzoylethan, 2,6-Dimethyl-4-tert.-butyl-4'-methoxydibenzoyl-methan, 2,4-Dimethyl-4'-methoxydibenzoylmethan.

Im besonderen bevorzugt wird das 2,4-Dimethyl-4'-methoxy-dibenzoyl-methan.

Die neuen Dibenzoyl-methan-Derivate können hergestellt werden, indem man alkylsubstituierte Acetophenon-Derivate der Formel

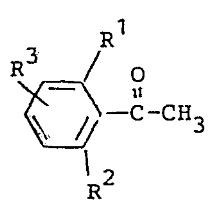
(III),

in der

$R^1$, $R^2$ und $R^3$ die oben genannte Bedeutung haben, mit einem Anissäureester der Formel

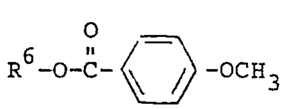
(IV),

in der

$R^6$ für einen niederen geradkettigen oder verzweigten Alkylrest steht,

in Gegenwart einer Base umsetzt.

Es ist auch möglich, die neuen Dibenzoyl-methan-Derivate herzustellen, indem man alkylsubstituierte Benzoesäureester der Formel

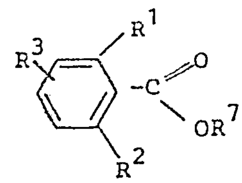
(V),

in der

$R^1$, $R^2$ und $R^3$ die oben genannte Bedeutung haben und

$R^7$ für einen niederen geradkettigen oder verzweigten Alkylrest steht,

mit p-Methoxy-aceptophenon in Gegenwart einer Base umsetzt.

Basen für die beiden erfindungsgemässen Herstellungsverfahren sind im wesentlichen starke Basen wie Alkali-alkoholate, wie Natrium- oder Kalium-methylat oder -ethylat, Alkalihydride, wie Natrium- oder Kaliumhydrid, oder Alkaliamide wie Natrium- oder Kaliumamid.

Die Umsetzungen werden im allgemeinen in einem aprotischen Lösungsmittel durchgeführt, das sich unter den Reaktionsbedingungen nicht verändert. Als Lösungsmittel seien beispielsweise genannt: Toluol, Benzol, Xylol, Diisopropylether.

Die beiden erfindungsgemässen Herstellungsverfahren werden im allgemeinen im Temperaturbereich von 60 bis 140° C, bevorzugt von 80 bis 120° C und bei Normaldruck durchgeführt.

Im allgemeinen stellt man die neuen Dibenzoylmethan-Derivate her, indem man die Base in einem Teil des Lösungsmittels auf die Reaktionstemperatur erwärmt und die Lösung der beiden Reaktanten im restlichen Lösungsmittel über einen längeren Zeitraum zutropft.

Die erfindungsgemässen Dibenzoyl-methan-Derivate liegen überwiegend in der Enolform vor. Die erfindungsgemässen Dibenzoyl-methan-Derivate können vorteilhaft als Wirkstoff in Lichtschutzmittel verwendet werden. Sie absorbieren die ultraviolette Strahlung der Sonne im sogenannten UV-A-Bereich (320 bis 400 nm). Die UV-A-Strahlung hat eine nachteilige Wirkung auf die menschliche Haut, da sie krankhafte Veränderungen der Haut, wie z.B. Lichtdermatosen hervorrufen kann und die Alterung der Haut beschleunigt.

Die Erfindung betrifft deshalb auch die Verwendung der erfindungsgemässen Dibenzoyl-methane in Lichtschutzmitteln.

Die Verwendung bestimmter Dibenzoylmethan-Derivate zum Schutz der Haut gegen UV-A-Strahlung ist bekannt. So beschreibt die DE-OS 2544180 Alkyl-dibenzoyl-methane der Formel

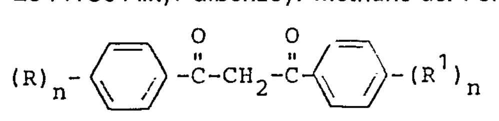
(VI),

in der

R und $R^1$ Alkylgruppen darstellen und

n für 0 bis 3 und

n' für 1 bis 3 steht.

Die Absorptionsmaxima dieser Filtersubstanzen liegen je nach Substitutionstyp zwischen 330 und 350 nm.

In der DE-OS 2945125 wird das 4-tert.-Butyl-4'-methoxydibenzoyl-methan beschrieben, dessen Absorptionsmaximum bei 355 nm liegt.

Gegenüber den in der DE-OS 2544180 beschriebenen Dibenzoyl-methanen haben die neuen erfindungsgemässen Dibenzoyl-methan-Derivate den überraschenden Vorteil eines deutlich höheren molaren Extinktionskoeffizienten. So hat beispielsweise das erfindungsgemässe 2,4-Dimethyl-4'-methoxy-dibenzoyl-methan einen molaren Extinktionskoeffizienten von 30.800, während die strukturell ähnlichste Verbindung der DE-OS 2544180, das 2,4-Dimethyl-dibenzoyl-methan, lediglich einen molaren Extinktionskoeffizienten von 24.200 aufweist.

Gegenüber dem 4-tert.-Butyl-4'-methoxy-dibenzoyl-methan der DE-OS 2945125 haben die erfindungsgemässen Dibenzoylmethane den Vorteil, dass ihr Absorptionsmaximum im kurzwelligeren Bereich liegt (zwischen 330 und 345 nm), so dass insbesondere die gefährlichere und energiereichere kurzwellige UV-A-Strahlung absorbiert wird. Ausserdem zeigen die erfindungsgemässen Dibenzoyl-methan-Derivate überraschenderweise eine bessere Öllöslichkeit als das 4-tert.-Butyl-4'-methoxy-dibenzoyl-methan und lassen sich besser in die für Sonnenschutzmittel üblichen kosmetischen Grundlagen einarbeiten. Die Licht- und Thermostabilität sowie die Hautverträglichkeit der erfindungsgemässen Dibenzoyl-methan-Derivate sind nahezu ausgezeichnet. Die erfindungsgemässen Dibenzoyl-methan-Derivate sind nahezu farblos und geruchlos.

Die Erfindung betrifft daher auch Lichtschutzmittel, die dadurch gekennzeichnet sind, dass sie die erfindungsgemässen Dibenzoyl-methan-Derivate enthalten.

Die Herstellung der erfindungsgemässen Lichtschutzmittel kann beispielsweise durch Einarbeitung der erfindungsgemässen Dibenzoyl-methan-Derivate in eine für Lichtschutzmittel übliche kosmetische Grundlage erfolgen. Die Einarbeitung erfolgt durch übliche Verteilungsmethoden wie beispielsweise Rühren oder Homogenisieren. Übliche kosmetische Grundlagen sind z.B. Cremes, Lotionen, Salben, Lösungen, Sprays und Milchen (G.H. Nowak, „Die kosmetischen Präparate", 2. Aufl., 1975).

Cremes für die erfindungsgemässen Lichtschutzmittel sind beispielsweise Emulsionen des Typs Wasser in Öl und Öl in Wasser.

Lotionen für die erfindungsgemässen Lichtschutzmittel sind beispielsweise alkoholisch-wässerige Öl- und Alkohol-Mischungen.

Salben für die erfindungsgemässen Lichtschutzmittel sind beispielsweise pharmazeutische Cremes.

Lösungen für die erfindungsgemässen Lichtschutzmittel sind beispielsweise Lösungen des Filters in kosmetischen Lösungsmitteln wie Ölen und Alkoholen.

Sprays für die erfindungsgemässen Lichtschutzmittel sind beispielsweise Lösungen in Verbindung mit einem Treibgas.

Milchen für die erfindungsgemässen Lichtschutzmittel sind flüssige stabile Emulsionen des Typs Wasser in Öl und Öl in Wasser.

Der Gehalt der erfindungsgemässen Dibenzoyl-methan-Derivate in Lichtschutzmitteln beträgt in Abhängigkeit von der kosmetischen Grundlage 1 bis 6 Gew.-%, vorzugsweise 2 bis 5 Gew.-%, bezogen auf die kosmetische Grundlage.

Für spezielle Verwendungen, z.B. medizinische Präparate, sind selbstverständlich auch höhere Dosierungen möglich.

Im allgemeinen ist es vorteilhaft, in einem Lichtschutzmittel, das ein UV-A-Filter enthält, zusätzlich eine Filtersubstanz für den UV-B-Bereich (290 bis 320 nm) zu verwenden. Als übliche UV-B-Filter seien beispielsweise die in der DE-OS 2945125 (Seite 4 unten bis Seite 5 unten) aufgezählten Verbindungen genannt. Besonders bevorzugt kombiniert man die neuen Dibenzoyl-methan-Derivate mit UV-B-Filtersubstanzen wie p-Methoxy-zimtsäure-(2-ethyl-hexyl)-ester oder p-Methoxy-zimtsäure-isoamylester.

Im allgemeinen beträgt in den erfindungsgemässen Lichtschutzmitteln das Verhältnis der erfindungsgemässen Dibenzoyl-methan-Derivaten zu den UV-B-Filtern 1:3 bis 2:1.

*Beispiel 1:*

Zu einer gerührten Suspension von 36 g (1,5 Mol) Natriumhydrid in 180 g trockenem Toluol tropft man bei 100° C unter Stickstoff innerhalb von 3 h eine Lösung von 148 g 2,4-Dimethylacetophenon (1,0 Mol) und 332 g Anissäuremethylester (2,0 Mol) in 180 g Toluol. Während der Dosierung entweicht Wasserstoff, und das Reaktionsgemisch wird dickflüssig. Nach beendeter Zugabe rührt man noch 3 h bei 100° C nach, kühlt dann auf Raumtemperatur ab und gibt 400 ml Wasser, 170 g konzentrierte Salzsäure und 800 ml Ethylacetat zu. Man filtriert die unlöslichen Bestandteile ab und trennt vom Filtrat die wässerige Phase ab. Die organische Phase wird mit Wasser gewaschen, vom Ethylacetat befreit und zur Wiedergewinnung des überschüssigen Anissäuremethylesters im Vakuum andestilliert. Man erhält 138 g nicht umgesetzten Anissäuremethylester. Der Rückstand wird aus einem Gemisch aus Isopropanol und Toluol umkristallisiert. Man erhält 198 g (70,2% d.Th.) 2,4-Dimethyl-4'-methoxy-dibenzoyl-methan.

Fp=48° C, UV-Spektrum: $\lambda$ max = 345 nm, E (1%, 1 cm) = 1090

*Beispiel 2:*

Analog Beispiel 1 erhält man aus 2,4-Diethylacetophenon und Anissäuremethylester in Gegenwart von Natriumhydrid das 2,4-Diethyl-4'-methoxy-dibenzoyl-methan.

Fp=38° C, UV-Spektrum: $\lambda$ max = 344 nm, E (1%, 1 cm) = 1020

*Beispiel 3:*

Analog Beispiel 1 erhält man aus 2,6-Dimethyl-4-tert.-butyl-acetophenon und Anissäuremethylester das 2,6-Dimethyl-4-tert.-butyl-4'-methoxy-dibenzoylmethan.
Fp=108° C, UV-Spektrum: λ max=333 nm, E (1%, 1 cm)=850

*Beispiel 4:*

Analog Beispiel 1 erhält man aus 2-Methyl-5-isopropylacetophenon und Anissäuremethylester in Gegenwart von Natriumhydrid das 2-Methyl-5-isopropyl-4'-methoxy-dibenzoylmethan.
Fp=74° C, UV-Spektrum: λ max=343 nm, E (1%, 1 cm)=980

*Beispiel 5:*

Analog Beispiel 1 erhält man aus 2-Methyl-5-tert.-butyl-acetophenon und Anissäuremethylester in Gegenwart von Natriumhydrid das 2-Methyl-5-tert.-butyl-4'-methoxy-dibenzoylmethan als viskoses Öl, das durch Säulenchromatographie an Kieselgel gereinigt wird.
UV-Spektrum: λ max=345 nm, E (1%, 1 cm)=760

*Beispiel 6:*

Analog Beispiel 1 erhält man aus 2,5-Dimethylacetophenon und Anissäuremethylester in Gegenwart von Natriumhydrid das 2,5-Dimethyl-4'-methoxy-dibenzoylmethan.
Fp=87° C, UV-Spektrum: λ max=342 nm, E (1%, 1 cm)=1010

*Sonnenschutzmilch (Emulsionstyp O/W)*

A. 3,50% Emulgator Mono-, Di- und Tri-(alkyltetraglykolether) o-Phosphorsäureester
1,50% Emulgator Stearinsäurepolyglycerinesteroxethylat
0,80% Cetylalkohol
3,00% Paraffinöl perliquidum
5,00% Isopropylmyristat
4,50% p-Methoxy-zimtsäure-(2-ethylhexyl)-ester
2,50% 2-Methyl-5-tert.-butyl-4'-methoxy-dibenzoylmethan
0,05% p-Hydroxybenzoesäurepropylester
B. 73,80% Wasser dest.
0,15% p-Hydroxybenzoesäuremethylester
3,00% Sorbitol 70%ige wässerige Lösung
0,20% Imidazolidinylharnstoffderivat
0,30% Carboxy-Vinyl-Polymerisat
1,20% Natriumhydroxid, 10%ige Lösung
0,50% Parfümöl

*Herstellungsvorschrift:*

Teil A: Bestandteile zusammengeben und auf 80-85° C bis zu einer klaren Schmelze erhitzen.

Teil B: Carboxy-Vinyl-Polymerisat im Wasser klumpenfrei dispergieren. Dann Sorbitol, p-Hydroxybenzoesäuremethylester und Imidazolidinylharnstoffderivat zugeben und auf 90° C erhitzen. Anschliessend mit Natriumhydroxid neutralisieren und Teil B in Teil A einrühren. Dann die Emulsion auf 40° C abrühren, das Parfümöl zugeben und weiter auf Raumtemperatur abrühren.

*Sonnenschutzöllotion*

43,50% Polyoxypropylen-(15)-stearylether
11,00% Isoproylmyristat
6,00% p-Methoxyzimtsäure-(2-ethylhexyl)-ester
3,00% 2-Methyl-5-isopropyl-4'-methoxydibenzoylmethan
35,00% Ethylalkohol 96 Vol.-% vergällt
1,00% Lanolin flüssig
0,50% Parfümöl

*Herstellungsvorschrift:*

2-Methyl-5-isopropyl-4'-methoxy-dibenzoylmethan in der Mischung aus Polyoxypropylen-(15)-stearylether, Isopropylmyristat und 2-Ethylhexyl-p-methoxycinnamate unter leichtem Erwärmen lösen (40-50° C). Dann die restlichen Bestandteile der Reihenfolge nach einrühren.

*Sonnenschutzöl*

15,00% Isopropylmyristat
20,00% Kokosöl, raffiniert
6,00% p-Methoxy-zimtsäure-isoamylester
4,00% 2,3-Dimethyl-4'-methoxy-dibenzoylmethan
53,50% Paraffinöl perliquidum
1,00% Lanolin flüssig
0,50% Parfümöl

*Herstellungsvorschrift:*

2,4-Dimethyl-4'-methoxy-dibenzoylmethan in der Mischung aus Isopropylmyristat, Kokosöl und Isoamyl-p-methoxycinnamat unter leichtem Erwärmen (40-50° C) lösen. Dann die restlichen Bestandteile der Reihenfolge nach einrühren.

*Sonnenschutzcreme (Emulsionstyp W/O)*

A. 20,00% Kombination nichtionogener Fettsäureester mehrwertiger Alkohole mit Wachsen und gereinigten, gesättigten Kohlenwasserstoffen
4,00% p-Methoxy-zimtsäure-isoamyl-ester
4,00% Paraffinöl perliquidum
3,00% Ölsäuredecylester
4,00% 2,4-Diethyl-4'-methoxy-dibenzoylmethan
0,15% p-Hydroxybenzoesäurepropylester
B. 58,30% Wasser dest.
0,15% p-Hydroxybenzoesäuremethylester
0,20% Imidazolidinylharnstoffderivat
0,50% Magnesiumsulfat·7 $H_2O$
5,00% Sorbitol, 70%ige wässerige Lösung
0,70% Parfümöl

*Herstellungsvorschrift:*

Teil A: Bestandteile zusammengeben und auf 85° C erhitzen.

Teil B: Die Wasserphase auf 90° C erhitzen und dann in Teil A einrühren. Bei einer Emulsionstemperatur von 40° C wird das Parfümöl der Emulsion zugegeben.

Anschliessend wird mittels Kolloidmühle oder Walzenstuhl homogenisiert.

## Patentansprüche

1. Dibenzoyl-methan-Derivate der Formel

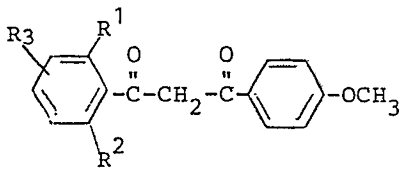

in der
$R^1$ Wasserstoff, Methyl oder Ethyl und
$R^2$ Methyl oder Ethyl bedeutet und
$R^3$ für einen niederen geradkettigen oder verzweigten Alkylrest steht.

2. Dibenzoyl-methan-Derivate nach Anspruch 1 der Formel

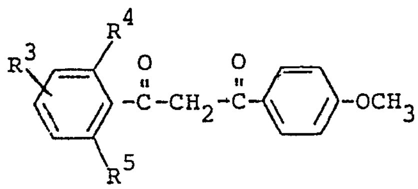

in der
$R^3$ für einen niederen geradkettigen oder verzweigten Alkylrest steht,
$R^4$ Wasserstoff oder Methyl und
$R^5$ Methyl bedeutet.

3. Verfahren zur Herstellung von Dibenzoyl-methan-Derivaten nach Anspruch 1, dadurch gekennzeichnet, dass man Alkyl-substituierte Acetophenon-Derivate der Formel

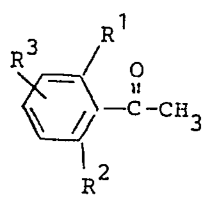

in der
$R^1$ Wasserstoff, Methyl oder Ethyl und
$R^2$ Methyl oder Ethyl bedeutet und
$R^3$ für einen niederen geradkettigen oder verzweigten Alkylrest steht,
mit einem Anissäureester der Formel

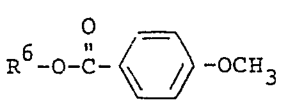

in der
$R^6$ für einen niederen geradkettigen oder verzweigten Alkylrest steht, in Gegenwart einer Base umsetzt.

4. Verfahren zur Herstellung von Dibenzoyl-methan-Derivaten nach Anspruch 1, dadurch gekennzeichnet, dass man Alkyl-substituierte Benzoesäureester der Formel

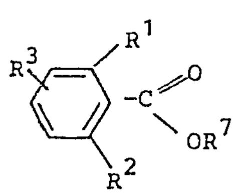

in der
$R^1$ Wasserstoff, Methyl oder Ethyl und
$R^2$ Methyl oder Ethyl bedeutet,
$R^3$ für einen niederen geradkettigen oder verzweigten Alkylrest steht und
$R^7$ für einen niederen geradkettigen oder verzweigten Alkylrest steht,
mit p-Methoxy-acetophenon in Gegenwart einer Base umsetzt.

5. Lichtschutzmittel, enthaltend Dibenzoyl-methan-Derivate nach Anspruch 1.

6. Lichtschutzmittel nach Anspruch 5, dadurch gekennzeichnet, dass es 1 bis 6 Gew.-% eines Dibenzoylmethan-Derivats, bezogen auf die kosmetische Grundlage, enthält.

7. Verwendung von Dibenzoyl-methan-Derivaten nach Anspruch 1 in Lichtschutzmitteln zum Schutz der Haut vor UV-Strahlen.

8. Verwendung nach Anspruch 7, dadurch gekennzeichnet, dass Dibenzoylmethan-Derivate in kosmetischen Präparaten enthalten sind.

## Claims

1. Dibenzoylmethane derivatives of the formula

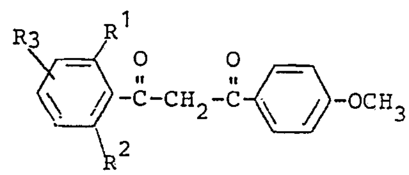

in which
$R^1$ denotes hydrogen, methyl or ethyl and
$R^2$ denotes methyl or ethyl and
$R^3$ represents a straight-chain or branched lower alkyl radical.

2. Dibenzoylmethane derivatives according to Claim 1, of the formula

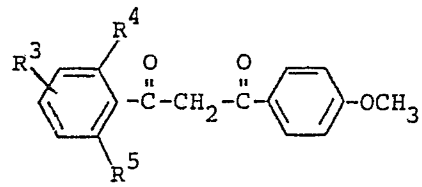

in which
$R^3$ represents a straight-chain or branched lower alkyl radical,
$R^4$ denotes hydrogen or methyl and
$R^5$ denotes methyl.

3. Process for the preparation of dibenzoylmethane derivatives according to Claim 1, characterised in that alkyl-substituted acetophenone derivatives of the formula

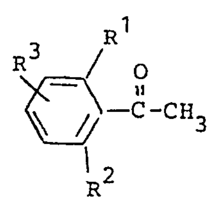

in which
$R^1$ denotes hydrogen, methyl or ethyl and

$R^2$ denotes methyl or ethyl and

$R^3$ represents a straight-chain or branched lower alkyl radical,

are reacted, in the presence of a base, with an anisic ester of the formula

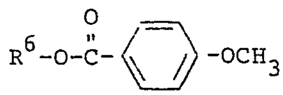

in which

$R^6$ represents a straight-chain or branched lower alkyl radical.

4. Process for the preparation of dibenzoylmethane derivatives according to Claim 1, characterised in that alkyl-substituted benzoic esters of the formula

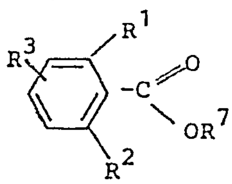

in which

$R^1$ denotes hydrogen, methyl or ethyl and

$R^2$ denotes methyl or ethyl,

$R^3$ represents a straight-chain or branched lower alkyl radical and

$R^7$ represents a straight-chain or branched lower alkyl radical,

are reacted, in the presence of a base, with p-methoxyacetophenone.

5. Sunscreen agent containing dibenzoylmethane derivatives according to Claim 1.

6. Sunscreen agent according to Claim 5, characterised in that it contains 1 to 6% by weight of a dibenzoylmethane derivative relative to the cosmetic base.

7. Use of dibenzoylmethane derivatives according to Claim 1 in sunscreen agents for protecting the skin from UV radiation.

8. Use according to Claim 7, characterised in that dibenzoylmethane derivatives are present in cosmetic products.

## Revendications

1. Dérivés de dibenzoylméthane de formule

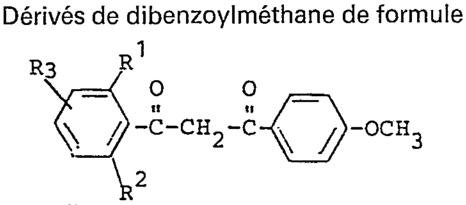

dans laquelle

$R^1$ représente l'hydrogène, le groupe méthyle ou le groupe éthyle et

$R^2$ est le groupe méthyle ou éthyle et

$R^3$ désigne un reste alkyle inférieur à chaîne droite ou ramifiée.

2. Dérivés de dibenzoylméthane suivant la revendication 1, de formule

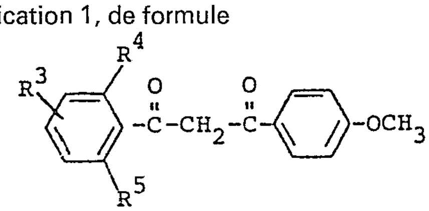

dans laquelle

$R^3$ représente un reste alkyle inférieur à chaîne droite ou ramifiée,

$R^4$ est l'hydrogène ou le groupe méthyle et

$R^5$ est le groupe méthyle.

3. Procédé de production de dérivés de dibenzoylméthane suivant la revendication 1, caractérisé en ce qu'on fait réagir des dérivés d'acétophénone à substituants alkyle de formule

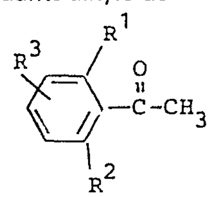

dans laquelle

$R^1$ est l'hydrogène, le groupe méthyle ou le groupe éthyle et

$R^2$ est le groupe méthyle ou éthyle et

$R^3$ est un reste alkyle inférieur à chaîne droite ou ramifiée,

avec un ester d'acide anisique de formule

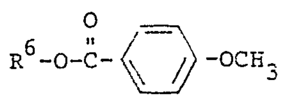

dans laquelle

$R^6$ est un reste alkyle inférieur à chaîne droite ou ramifiée, en présence d'une base.

4. Procédé de production de dérivés de dibenzoylméthane suivant la revendication 1, caractérisé en ce qu'on fait réagir des esters d'acide benzoïque à substituants alkyle de formule

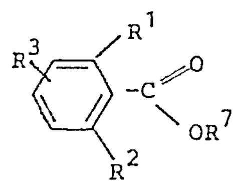

dans laquelle

$R^1$ est l'hydrogène, le groupe méthyle ou le groupe éthyle et

$R^2$ est le groupe méthyle ou éthyle,

$R^3$ est un reste alkyle inférieur à chaîne droite ou ramifiée et

$R^7$ est un reste alkyle inférieur à chaîne droite ou ramifiée,

avec la p-méthoxyacétophénone en présence d'une base.

5. Composition de protection solaire, contenant des dérivés de dibenzoylméthane suivant la revendication 1.

6. Composition de protection solaire suivant la revendication 5, caractérisée en ce qu'elle contient 1 à 6% en poids d'un dérivé de dibenzoylméthane, par rapport à la base cosmétique.

7. Utilisation de dérivés de dibenzoylméthane suivant la revendication 1 dans des compositions de protection solaire, pour protéger la peau contre l'action des rayons ultraviolets.

8. Utilisation suivant la revendication 7, caractérisée en ce que des dérivés de dibenzoylméthane sont contenus dans des préparations cosmétiques.